# EUROPEAN PATENT APPLICATION

(11) **EP 4 389 036 A1**
(43) Date of publication of application: **26.06.2024**
(21) Application number: 23217963.0
(22) Date of filing: 19.12.2023
(51) Int. Cl.: A61B 18/08, A61B 18/14, A61B 18/00, A61B 5/00, A61B 18/12

(54) **MULTI-ELECTRODE BASKET END EFFECTOR OF A CATHETER**

(30) Priority: 20.12.2022 US 202263476275 P; 10.11.2023 US 202318506529
(71) Applicant: Biosense Webster (Israel) Ltd., 2066717 Yokneam (IL)
(72) Inventor: VAN NIEKERK, Pieter Emmelius, Irvine, 92618 (US); ROSEBERRY, Jacob, Irvine, 92618 (US); PATEL, Amar, Irvine, 92618 (US); JIMENEZ, Jose, Irvine, 92618 (US); NGUYEN, Thanh, Irvine, 92618 (US); RORICK, Kevin, Irvine, 92618 (US); XU, Guo, Irvine, 92618 (US); SUAREZ, Paul, Irvine, 92618 (US)
(74) Representative: Carpmaels & Ransford LLP

(57) **Abstract**

Examples presented herein illustrate various end effector designs with multiple spines that can expand from a collapsed configuration as the end effector traverses vasculature to an expanded configuration when the end effector is at a treatment site. In some examples, the end effector includes three frame loops which each include a pair of spines. At least one of the three frame-loops can have a bend at a distal end of the end effector so that the spines of the frame loop are not directly opposite each other with respect to the longitudinal axis. In some examples, the end effector includes an inner and an outer frame, each having multiple spines and configured such that one or both of the frames can rotate from an aligned configuration to an unaligned configuration.

## Description

### CROSS-REFERENCE TO RELATED APPLICATIONS

This application claims the benefit of prior filed U.S. Provisional Patent Application No. 63/476,275 filed on December 20, 2022, which is hereby incorporated by reference as set forth in full herein.

### FIELD

The present invention relates generally to medical devices, and in particular to multi-electrode catheters for mapping and/or ablation of tissue within a patient.

### BACKGROUND

Cardiac arrhythmias, such as atrial fibrillation (AF), occur when regions of cardiac tissue abnormally conduct electric signals to adjacent tissue. This disrupts the normal cardiac cycle and causes asynchronous rhythm. Certain procedures exist for treating arrhythmia, including surgically disrupting the origin of the signals causing the arrhythmia and disrupting the conducting pathway for such signals. By selectively ablating cardiac tissue by application of energy via a catheter, it is sometimes possible to cease or modify the propagation of unwanted electrical signals from one portion of the heart to another.

Many current ablation approaches in the art tend to utilize radiofrequency (RF) electrical energy to heat tissue. RF ablation can have certain rare drawbacks due to operator's skill, such as heightened risk of thermal cell injury which can lead to tissue charring, burning, steam pop, phrenic nerve palsy, pulmonary vein stenosis, and esophageal fistula. Cryoablation is an alternative approach to RF ablation that can reduce some thermal risks associated with RF ablation but may present tissue damage due to the very low temperature nature of such devices. Maneuvering cryoablation devices and selectively applying cryoablation, however, is generally more challenging compared to RF ablation; therefore, cryoablation is not viable in certain anatomical geometries which may be reached by electrical ablation devices.

Some ablation approaches use irreversible electroporation (IRE) to ablate cardiac tissue using nonthermal ablation methods. IRE delivers short pulses of high voltage to tissues and generates an unrecoverable permeabilization of cell membranes. Delivery of IRE energy to tissues using multi-electrode catheters was previously proposed in the patent literature. Examples of systems and devices configured for IRE ablation are disclosed in U.S. Patent Pub. No. 2021/0169550A1, 2021/0169567A1, 2021/0169568A1, 2021/0161592A1, 2021/0196372A1, 2021/0177503A1, and 2021/0186604A1, each of which are incorporated herein by reference and attached to the Appendix of priority patent Application No. 63/476,275.

Regions of cardiac tissue can be mapped by a catheter to identify the abnormal electrical signals. The same or different catheter can be used to perform ablation. Some example catheters include a number of spines with electrodes positioned thereon. The electrodes are generally attached to the spines and secured in place by soldering, welding, or using an adhesive. Furthermore, multiple linear spines are generally assembled together by attaching both ends of the linear spines to a tubular shaft (e.g., a pusher tube) to form a spherical basket. Due to the small size of the spines and the electrodes, however, adhering the electrodes to the spines and then forming a spherical basket from the multiple linear spines can be a difficult task, increasing the manufacturing time and cost and the chances that the electrode fails due to an improper bond or misalignment. What is needed, therefore, are devices and methods of forming a basket assembly that can help to reduce the time required for manufacturing the basket assembly, alternative catheter geometries, and support frames that can support alternative electrode shapes and sizes.

### SUMMARY

Examples presented herein illustrate various end effector designs with multiple spines that can expand from a collapsed configuration as the end effector traverses vasculature to an expanded configuration when the end effector is at a treatment site. In some examples, the end effector includes three frame loops which each include a pair of spines. At least one of the three frame-loops can have a bend at a distal end of the end effector so that the spines of the frame loop are not directly opposite each other with respect to the longitudinal axis. In some examples, the end effector includes an inner and an outer frame, each having multiple spines and configured such that one or both of the frames can rotate from an aligned configuration to an unaligned configuration. In the aligned configuration, electrodes of the inner frame are inhibited by the outer frame from contacting tissue. In the unaligned configuration, electrodes of the inner frame are positioned to be able to contact tissue.

An example end effector of a catheter can include a plurality of spines, a first frame loop, a second frame loop, a third frame loop, and one or more electrodes. The plurality of spines can be configured to expand away from a longitudinal axis of the end effector to form a basket shape. The first frame loop can include a first pair of spines of the plurality of spines such that spines of the first pair of spines are disposed across from each other with respect to the longitudinal axis. The second frame loop is distinct from the first frame loop and can include second pair of spines of the plurality of spines such that spines of the second pair of spines are disposed on a first side of the first frame loop. The third frame loop is distinct from the first frame loop and the second frame loop and includes a third pair of spines of the plurality of spines such that spines of the third pair of spines are disposed on a second side of the first frame loop, the second side being opposite the first side. The one or more electrodes of the example end effector are coupled to the plurality of spines.

The first frame loop includes a distal portion approximate a distal end of the end effector and traversing the longitudinal axis.

Each of the second frame loop and the third frame loop respectively include an angled portion approximate a distal end of the end effector. The angled portion of each of the second and third frame loop can be coupled to the first frame loop.

Spines of the plurality of spines can be disposed symmetrically about the longitudinal axis.

Spines of the first pair of spines, the second pair of spines, and the third pair of spines can be disposed symmetrically about the longitudinal axis.

The first pair of spines can be disposed approximately 180° from each other with respect to an imaginary circle about the longitudinal axis. The second pair of spines can be disposed approximately 60° from each other with respect to the imaginary circle. The third pair of spines can be disposed approximately 60° from each other with respect to the imaginary circle.

The first frame loop can include a distal portion at a distal end of the end effector. The distal portion can traverse the longitudinal axis. Each of the second frame loop and the third frame loop can respectively include an angled portion approximate a distal end of the end effector such that each angled portion overlaps the distal portion of the first frame loop.

The first frame loop can include a distal portion at a distal end of the end effector and traversing the longitudinal axis. Each of the second frame loop and the third frame loop can include an angled portion approximate a distal end of the end effector such that each angled portion abuts the distal portion of the first frame loop.

The second frame loop can be disposed entirely on the first side of the first frame loop. The third frame loop can be disposed entirely on the second side of the first frame loop.

The end effector can further include a retainer which couples the first frame loop, the second frame loop, and the third frame loop approximate a distal end of the end effector.

Each electrode of the one or more electrodes can define a lumen through the electrode so that each spine of the plurality spines extends through the lumen of each of the one or more electrodes.

The basket shape can be approximately spherical.

The basket shape can be approximately oblate-spheroid.

The one or more electrodes can be configured to deliver electrical pulses for irreversible electroporation, the pulses including a peak voltage of at least 900 volts (V).

Another example end effector of a catheter can include a plurality of spines, a first frame loop, a second frame loop, a third frame loop, and one or more electrodes. The plurality of spines can be configured to expand from a longitudinal axis of the end effector to form a basket shape. The first frame loop can include a first pair of spines of the plurality of spines and a first angled portion approximate a distal end of the end effector. The second frame loop is distinct from the first frame loop and includes a second pair of spines of the plurality of spines and a second angled portion approximate the distal end of the end effector such that the second angle portion overlaps the first angled portion, an inner spine of the first pair of spines is positioned between spines of the second pair of spines, and an inner spine of the second pair of spines is positioned between spines the first pair of spines. The third frame loop is distinct from the first frame loop and the second frame loop and includes a third pair of spines of the plurality of spines and a third angled portion approximate the distal end of the end effector such that spines of the third pair of spines are each disposed between outer spines of the first pair of spines and the second pair of spines. The one or more electrodes are coupled to the plurality of spines.

Spines of the plurality of spines can be disposed symmetrically about the longitudinal axis.

Spines of the first pair of spines, the second pair of spines, and the third pair of spines can be disposed symmetrically about the longitudinal axis.

The first pair of spines can be disposed approximately 120° from each other with respect to an imaginary circle about the longitudinal axis. The second pair of spines can be disposed approximately 120° from each other with respect to the imaginary circle. The third pair of spines being disposed approximately 60° from each other with respect to the imaginary circle.

The outer spine of the first pair of spines can be disposed opposite the outer spine of the second pair of spines with respect to the longitudinal axis.

The first angled portion and/or the second angled portion can overlap the third angled portion approximate the distal end of the end effector.

Each electrode of the one or more electrodes can define a lumen through the electrode so that each spine of the plurality spines extends through the lumen of each of the one or more electrodes.

The basket shape can be approximately spherical.

The basket shape can be approximately oblate-spheroid.

The one or more electrodes can be configured to deliver electrical pulses for irreversible electroporation, the pulses including a peak voltage of at least 900 volts (V).

Another example end effector of a catheter can include an outer electrode assembly and an inner electrode assembly. The outer electrode assembly can include a first plurality of spines and a first plurality of electrodes. The first plurality of spines can be configured to expand from a longitudinal axis of the end effector to form a basket shape. The first plurality of electrodes can be coupled to each of the first plurality of spines. The inner electrode assembly can include a second plurality of electrodes. The inner electrode assembly can be configured to move between a non-contacting configuration and a contacting configuration such that in the non-contacting configuration, the second plurality of electrodes are inhibited, by the outer electrode assembly, from contacting tissue, and such that in the contacting configuration, the second plurality of electrodes are positioned to contact tissue.

The outer electrode assembly can include a first unitary tripodic structure such that the first plurality of spines has exactly three spines. The inner electrode assembly can include a second unitary tripodic structure such that the second plurality of spines has exactly three spines. The second unitary tripodic structure can be rotatable to be in alignment with the first unitary tripodic structure in the non-contacting configuration and can be rotated to be out of alignment with first unitary tripodic structure in the contacting configuration. Each tripodic structure can be formed from a respective planar sheet of material that includes three linear spines converging at a respective central spine intersection. Each spine of each tripodic structure can include a respective end disposed at a proximal end of the end effector. The central spine intersection of each tripodic structure can be positioned on the longitudinal axis at a distal end of the end effector.

The inner electrode assembly can include a second plurality of spines. The second plurality of electrodes can be coupled to the second plurality of spines. The second plurality of spines can be in alignment with the first plurality of spines in the non-contacting configuration. The second plurality of spines being out of alignment with the first plurality of spines in the contacting configuration.

Each electrode of the first plurality of electrodes and of the second plurality of electrodes can define a lumen through the electrode so that each spine of the plurality spines extends through the lumen of each of the one or more electrodes.

The basket shape can be approximately spherical.

The basket shape can be approximately oblate-spheroid.

Electrodes of the first plurality of electrodes can be configured to deliver electrical pulses for irreversible electroporation. The pulses can have a peak voltage of at least 900 volts (V).

Electrodes of the second plurality of electrodes can be configured to map cardiac electrical signals through tissue.

Another example end effector can include an expandable basket assembly including a first unitary structure, a second unitary structure, and a plurality of electrodes. The first unitary structure can include four spines and can be formed from a planar sheet of material that includes four linear spines converging at a central spine intersection. The second unitary structure is distinct from the first unitary structure and can include at least two spines. The plurality of electrodes can be coupled to each spine of the first unitary structure and the second unitary structure. The spines of the first unitary structure and the second unitary structure can be configured to expand from a longitudinal axis of the end effector to collectively form a basket shape.

The secondary unitary structure can be formed from a planar sheet of material that includes the at least two spines converging at a central spine intersection. Each spine of each unitary structure can include a respective connected end disposed at a proximal end of the end effector. The central spine intersection of each unitary structure can be positioned on the longitudinal axis at a distal end of the end effector.

The second unitary structure can be formed from a tube of material that includes the at least two spines joined at one end by a ring.

The at least two spines can have exactly four spines. Alternatively, the at least two spines can have exactly three spines. Alternatively, the at least two spines can have exactly two spines.

The second unitary structure can include a loop extending across the central spine intersection of the first unitary structure such that two spines of the first unitary structure are disposed on a first side of the loop and two spines of the first unitary structure are disposed on a second side of the loop.

The spines of the first unitary structure and the second unitary structure can be positioned symmetrically about the longitudinal axis.

Each electrode of the plurality of electrodes can define a lumen through the electrode so that each spine of the plurality spines extends through the lumen of each of the one or more electrodes.

The basket shape can be approximately spherical.

The basket shape can be approximately oblate-spheroid.

The one or more electrodes can be configured to deliver electrical pulses for irreversible electroporation, the pulses including a peak voltage of at least 900 volts (V).

### BRIEF DESCRIPTION OF THE DRAWINGS

The above and further aspects of this invention are further discussed with reference to the following description in conjunction with the accompanying drawings, in which like numerals indicate like structural elements and features in various figures. The drawings are not necessarily to scale, emphasis instead being placed upon illustrating principles of the invention. The figures depict one or more implementations of the inventive devices, by way of example only, not by way of limitation.
Figure 1 is an illustration of an example catheter-based electrophysiology mapping and ablation system according to aspects of the present invention.
Figure 2A is an illustration of a first example basket assembly having three frame loops according to aspects of the present invention.
Figure 2B is an illustration of a distal end view of the first example basket assembly according to aspects of the present invention.
Figure 3A is an illustration of a second example basket assembly having three frame loops according to aspects of the present invention.
Figure 3B is an illustration of a distal end view of the second example basket assembly according to aspects of the present invention.
Figure 4A is an illustration of a third example basket assembly having three frame loops according to aspects of the present invention.
Figure 4B is an illustration of a distal end view of the third example basket assembly according to aspects of the present invention.
Figure 5A is an illustration of a fourth example basket assembly having an inner electrode assembly in a contacting configuration according to aspects of the present invention.
Figure 5B is an illustration of a portion of the fourth example basket assembly with the inner electrode assembly in a non-contacting configuration according to aspects of the present invention.
Figure 6A is an illustration of the arrangement of electrodes of the fourth example basket assembly with the inner electrode assembly in the non-contacting configuration according to aspects of the present invention.
Figure 6B is an illustration of the arrangement of electrodes of the fourth example basket assembly with the inner electrode assembly in the contacting configuration according to aspects of the present invention.
Figure 6C illustrates an exemplary basket made from two separate tubular stocks indicated as 410 and 420 with different outer diameters according to aspects of the present invention.
Figure 6D illustrates a cross-sectional view of the smaller tubular stock 420 (from Fig. 6C) with three separate spines according to aspects of the present invention.
Figure 6E illustrates a cross-sectional view of the larger tubular stock 410 (from Fig. 6C) with three separate spines according to aspects of the present invention.
Figure 7A is an illustration of a first unitary structure of a fifth example basket assembly according to aspects of the present invention.
Figure 7B is an illustration of a second unitary structure of the fifth example basket assembly according to aspects of the present invention.
Figure 7C is an illustration of the structures in Figure 7A and 7B assembled to form the fifth example basket assembly according to aspects of the present invention.
Figure 8A is an illustration of a first unitary structure of a sixth example basket assembly according to aspects of the present invention.
Figure 8B is an illustration of a second unitary structure of the sixth example basket assembly according to aspects of the present invention.
Figure 8C is an illustration of the structures in Figure 8A and 8B assembled to form the sixth example basket assembly according to aspects of the present invention.
Figure 9A is an illustration of a first unitary structure of a seventh example basket assembly according to aspects of the present invention.
Figure 9B is an illustration of a second unitary structure of the seventh example basket assembly according to aspects of the present invention.
Figure 9C is an illustration of the structures in Figure 9A and 9B assembled to form the seventh example basket assembly according to aspects of the present invention.
Figure 10A is an illustration of a first unitary structure of an eighth example basket assembly according to aspects of the present invention.
Figure 10B is an illustration of a second unitary structure of the eighth example basket assembly according to aspects of the present invention.
Figure 10C is an illustration of the structures in Figure 10A and 10B assembled to form the eighth example basket assembly according to aspects of the present invention.
Figure 11 is an illustration of an electrode according to aspects of the present invention.
Figure 12A is an illustration of a frame loop with an approximately circular cross-section according to aspects of the present invention.
Figure 12B is an illustration of a frame loop with an oblate cross-section according to aspects of the present invention.

### DETAILED DESCRIPTION

The following detailed description should be read with reference to the drawings, which depict selected embodiments and are not intended to limit the scope of the invention. The detailed description illustrates by way of example, not by way of limitation, the principles of the invention. This description will clearly enable one skilled in the art to make and use the invention, and describes several embodiments, adaptations, variations, alternatives and uses of the invention, including what is presently believed to be the best mode of carrying out the invention. Any one or more of the teachings, expressions, versions, examples, etc. described herein may be combined with any one or more of the other teachings, expressions, versions, examples, etc. that are described herein. The following-described teachings, expressions, versions, examples, etc. should therefore not be viewed in isolation relative to each other. Various suitable ways in which the teachings herein may be combined will be readily apparent to those skilled in the pertinent art in view of the teachings herein. Such modifications and variations are intended to be included within the scope of the claims.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±10% of the recited value, e.g. "about 90%" may refer to the range of values from 81% to 99%.

In addition, as used herein, the terms "patient," "host," "user," and "subject" refer to any human or animal subject and are not intended to limit the systems or methods to human use, although use of the subject invention in a human patient represents a preferred embodiment. As well, the term "proximal" indicates a location closer to the operator whereas "distal" indicates a location further away to the operator or physician.

As used herein, the term "proximal" indicates a location closer to the operator or physician whereas "distal" indicates a location further away to the operator or physician.

As used herein, "operator" can include a doctor, surgeon, technician, scientist, or any other individual or delivery instrumentation associated with delivery of a multi-electrode catheter for the treatment of drug refractory atrial fibrillation to a subject.

As used herein, the term "ablate" or "ablation", as it relates to the devices and corresponding systems of this disclosure, refers to components and structural features configured to reduce or prevent the generation of erratic cardiac signals in the cells by utilizing non-thermal energy, such as irreversible electroporation (IRE), referred throughout this disclosure interchangeably as pulsed electric field (PEF) and pulsed field ablation (PFA). Ablating or ablation as it relates to the devices and corresponding systems of this disclosure is used throughout this disclosure in reference to non-thermal ablation of cardiac tissue for certain conditions including, but not limited to, arrhythmias, atrial flutter ablation, pulmonary vein isolation, supraventricular tachycardia ablation, and ventricular tachycardia ablation. The term "ablate" or "ablation" also includes known methods, devices, and systems to achieve various forms of bodily tissue ablation, including thermal ablation, as understood by a person skilled in the relevant art.

As discussed herein, the terms "bipolar", "unipolar", and "monopolar" when used to refer to ablation schemes describe ablation schemes which differ with respect to electrical current path and electric field distribution. "Bipolar" refers to ablation scheme utilizing a current path between two electrodes that are both positioned at a treatment site; current density and electric flux density is typically approximately equal at each of the two electrodes. "Unipolar" and "monopolar" are used interchangeably herein to refer to ablation scheme utilizing a current path between two electrodes where one electrode including a high current density and high electric flux density is positioned at a treatment site, and a second electrode including comparatively lower current density and lower electric flux density is positioned remotely from the treatment site.

As discussed herein, the terms "tubular" and "tube" are to be construed broadly and are not limited to a structure that is a right cylinder or strictly circumferential in cross-section or of a uniform cross-section throughout its length. For example, the tubular structures are generally illustrated as a substantially right cylindrical structure. However, the tubular structures may have a tapered or curved outer surface without departing from the scope of the present disclosure.

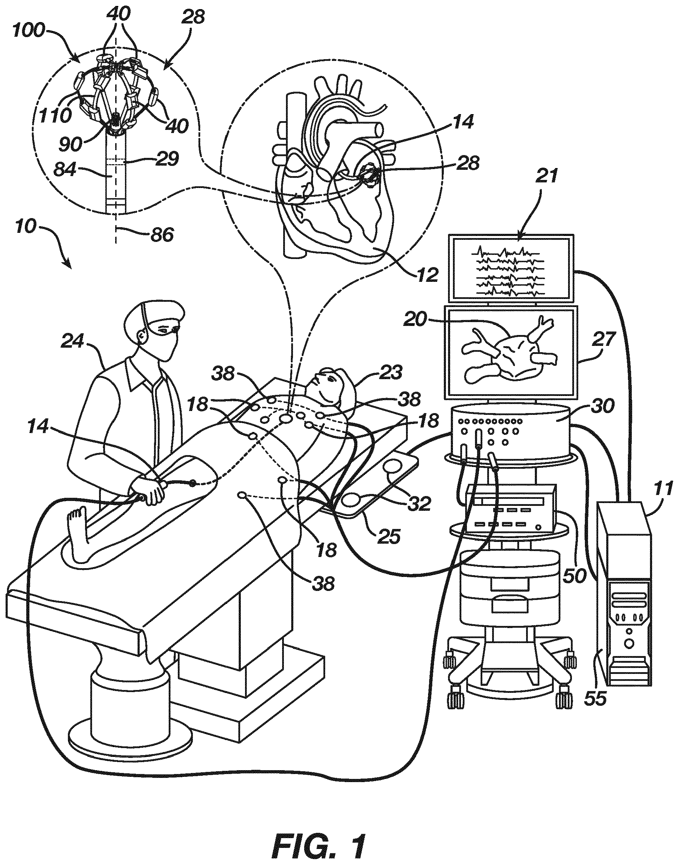

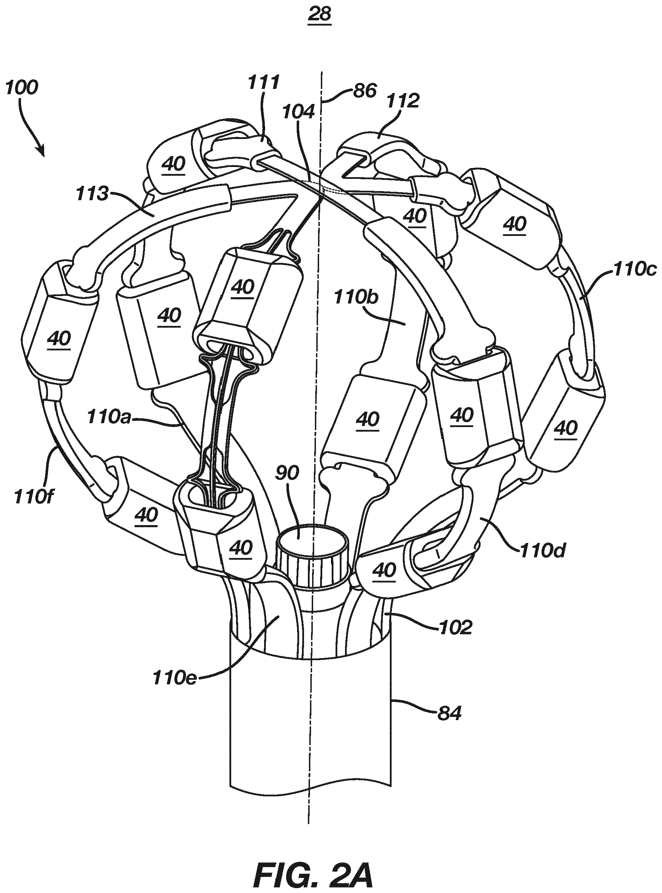

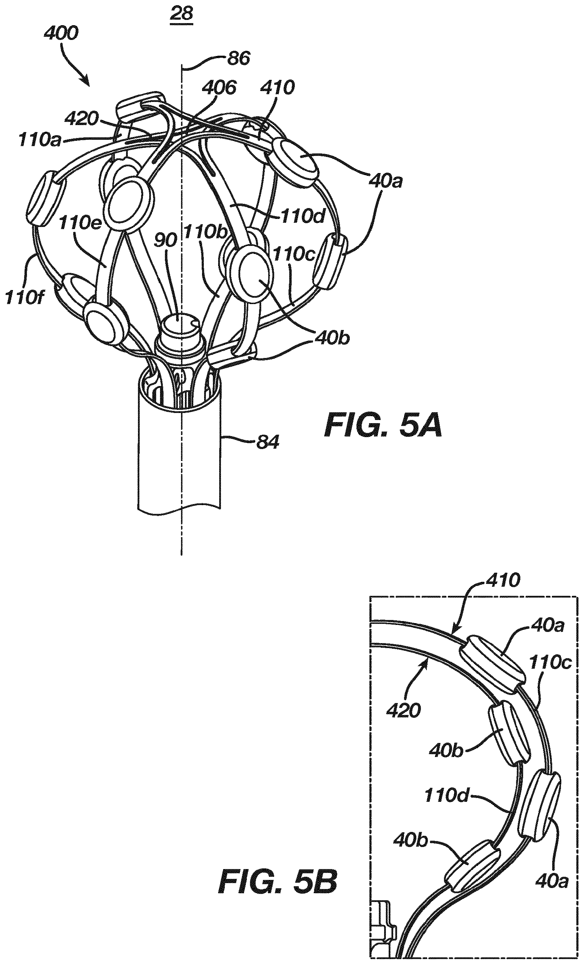

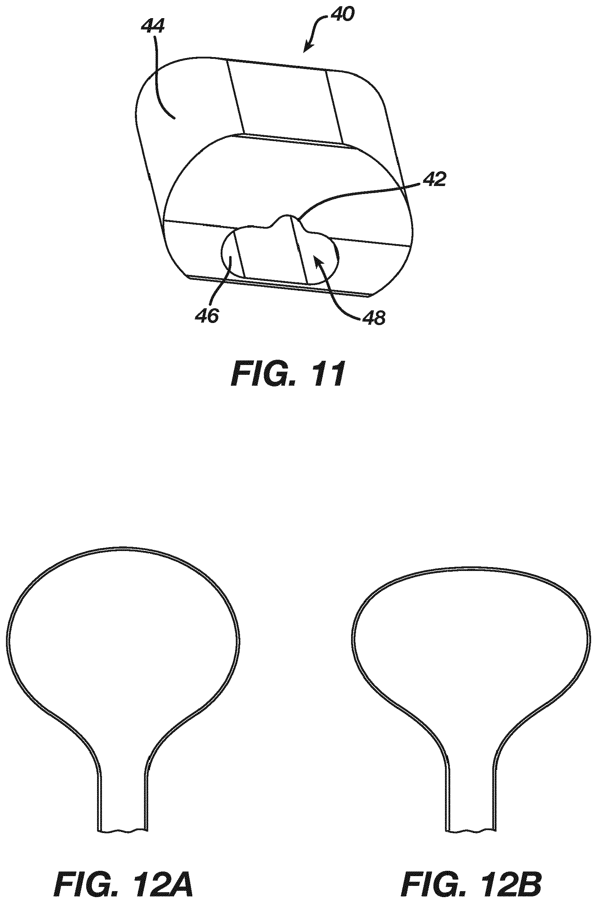

Alternative basket assembly configurations are presented herein. Figure 1 illustrates a catheter-based electrophysiology mapping and ablation system 10 including an example catheter 14. The example catheter 14 has electrodes 40 supported by spines 110 of a support frame assembly. The support frame assembly can have numerous configurations, including those illustrated in Figures 2A through 10C, where: first, second, and third example basket assemblies 100, 200, 300 illustrated in Figures 2A through 4B each include at least one frame loop with a bend at a distal end of the support frame assembly; a fourth example basket assembly 400 illustrated in Figures 5A through 6B has an inner and outer support frame which can cause an inner electrode assembly to be movable between a contacting configuration and a non-contacting configuration; fifth, sixth, and seventh example basket assemblies 500, 600, 700 each have a first unitary structure having four spines and a second unitary structure having at least two spines; and an eighth example basket assembly 800 has a combination of a planar tripodic structure and a tubular tripodic structure. Compatible features of each of the example basket assemblies 100, 200, 300, 400, 500, 600, 700, 800 are combinable as disclosed here and also as understood by a person skilled in the pertinent art. Each of the example basket assemblies 100, 200, 300, 400, 500, 600, 700, 800 can include electrodes such as the example electrode 40 illustrated in Figure 11. The profile shape of the support frame of any of the example basket assemblies 100, 200, 300, 400, 500, 600, 700, 800 can be approximately circular (thereby forming an approximately spherical shaped basket) as illustrated in Figure 12A or can be oblate (thereby forming an approximately oblate-spheroid shaped basket) as illustrated in Figure 12B. Examples are expounded upon in relation to the figures below.

Figure 1 is an illustration showing an example catheter-based electrophysiology mapping and ablation system 10. The system 10 includes multiple catheters, which are percutaneously inserted by a physician 24 through the patient's vascular system into a chamber or vascular structure of a heart 12. Typically, a delivery sheath catheter is inserted into the left or right atrium near a desired location in the heart 12. Thereafter, a plurality of catheters can be inserted into the delivery sheath catheter so as to arrive at the desired location. The plurality of catheters may include catheters dedicated for sensing Intracardiac Electrogram (IEGM) signals, catheters dedicated for ablating and/or catheters dedicated for both sensing and ablating. An example catheter 14 that is configured for sensing IEGM is illustrated herein. The physician 24 brings a distal tip 28 of the catheter 14 into contact with the heart wall for sensing a target site in the heart 12. For ablation, the physician 24 would similarly bring a distal end of an ablation catheter to a target site for ablating.

The illustrated catheter 14 is an exemplary catheter that includes one and preferably multiple electrodes 40 optionally distributed over a plurality of spines 110 of a basket assembly 100 which forms an end effector at distal tip 28 and configured to sense the IEGM signals and/or provide ablation signals. The basket assembly 100 illustrated in Figure 1 is compatible with every feature of the basket assemblies 100, 200, 300, 400, 500, 600, 700, 800 illustrated in Figures 2A through 10C and variations thereof as disclosed herein. Therefore catheter 14 can be modified to include any of the features related to the example basket assemblies 100, 200, 300, 400, 500, 600, 700, 800. Features of a given example basket assembly 100, 200, 300, 400, 500, 600, 700, 800 are inter-compatible within the given example. The overall basket shape can be approximately spherical (Figure 12A) or approximately oblate-spheroid (Figure 12B) for any of the example basket assemblies 100, 200, 300, 400, 500, 600, 700, 800.

The spines 110 can be collapsed toward the longitudinal axis 86 so that the basket assembly 100 end effector (referred herein after as "basket assembly") can be delivered through a sheath or intermediate catheter to a treatment site. The basket assembly 100 can be configured to expand to the illustrated basket shape when deployed, having an approximately spherical shape or an approximately oblate-spheroid shape as illustrated in profile in Figures 12A and 12B. Preferably, the support frame assembly 100 is self-expandable upon exiting the intermediate catheter or sheath, and may include nitinol or other shape memory material suitable to facilitate self-expansion and biocompatibility.

Proximal ends of each spine 110 are coupled together within the shaft 84 near a proximal end of the basket assembly 100 and a distal end of the shaft 84. The catheter 14 can include a spine retention hub 90 that extends longitudinally through the distal end of the shaft 84. The spine retention hub 90 can include a cylindrical member configured to affix proximal ends of the spines 110 within the shaft 84. The spine retention hub 90 may include irrigation openings and/or an electrode.

Each spine 110 can include a resilient support frame including a suitable biocompatible material to provide structural support and an insulative jacket, sleeve, or other structure electrically insulating the electrodes 40 from the support frame material. Each spine 110 can also include electrical conductors such as wires and/or flex circuits in electrical communication with the electrodes 40 to provide electrical communication between the electrodes 40 and other components of the system 10 (e.g. patient interface unit 30) to facilitate navigation, mapping, and/or ablation.

The catheter 14 may additionally include a position sensor 29 embedded in or near distal tip 28 for tracking position and orientation of distal tip 28. Optionally and preferably, position sensor 29 is a magnetic based position sensor including three magnetic coils for sensing three-dimensional (3D) position and orientation. A magnetic based position sensor 29 may be operated together with a location pad 25 including a plurality of magnetic coils 32 configured to generate magnetic fields in a predefined working volume. Real time position of a distal tip 28 of the catheter 14 may be tracked based on magnetic fields generated with a location pad 25 and sensed by a magnetic based position sensor 29. Details of the magnetic based position sensing technology are described in U.S. Patent Nos. 5,391,199; 5,443,489; 5,558,091; 6,172,499; 6,239,724; 6,332,089; 6,484,118; 6,618,612; 6,690,963; 6,788,967; and 6,892,091 incorporated by reference herein and attached to the Appendix of priority patent Application No. 63/476,275.

The system 10 includes one or more electrode patches 38 positioned for skin contact on the patient 23 to establish location reference for location pad 25 as well as impedance-based tracking of electrodes 40. For impedance-based tracking, electrical current is directed toward electrodes 40 and sensed at electrode skin patches 38 so that the location of each electrode can be triangulated via the electrode patches 38. Details of the impedance-based location tracking technology are described in U.S. Patent Nos. 7,536,218; 7,756,576; 7,848,787; 7,869,865; and 8,456,182 incorporated by reference herein and attached to the Appendix of priority patent Application No. 63/476,275.

A recorder 11 displays electrograms 21 captured with body surface ECG electrodes 18 and intracardiac electrograms (IEGM) captured with electrodes 40 of the catheter 14. The recorder 11 may include pacing capability for pacing the heart rhythm and/or may be electrically connected to a standalone pacer.

The system 10 can include an ablation energy generator 50 that is adapted to conduct ablative energy to one or more of electrodes at a distal tip of a catheter configured for ablating. Energy produced by the ablation energy generator 50 may include, but is not limited to, radiofrequency (RF) energy or pulsed-field ablation (PFA) energy, including monopolar or bipolar high-voltage DC pulses as may be used to effect irreversible electroporation (IRE), or combinations thereof. For instance, the electrodes 40 can be configured to deliver a peak voltage of at least 900 volts (V) between electrodes 40 to achieve IRE.

A patient interface unit (PIU) 30 is an interface configured to establish electrical communication between catheters, electrophysiological equipment, power supply and a workstation 55 for controlling operation of system 10. Electrophysiological equipment of the system 10 may include for example, multiple catheters, a location pad 25, body surface ECG electrodes 18, electrode patches 38, an ablation energy generator 50, and a recorder 11. Optionally and preferably, the PIU 30 includes processing capability for implementing real-time computations of location of the catheters and for performing ECG calculations.

The workstation 55 includes memory, processor unit with memory or storage with appropriate operating software loaded therein, and user interface capability. The workstation 55 can be configured to provide multiple functions, optionally including (1) modeling the endocardial anatomy in three-dimensions (3D) and rendering the model or an anatomical map 20 for display on a display device 27; (2) displaying on the display device 27 activation sequences (or other data) compiled from recorded electrograms 21 in representative visual indicia or imagery superimposed on the rendered anatomical map 20; (3) displaying real-time location and orientation of multiple catheters within the heart chamber; and (4) displaying on the display device 27 sites of interest such as places where ablation energy has been applied. One commercial product embodying elements of the system 10 is available as the CARTO^{™} 3 System, available from Biosense Webster, Inc., 31A Technology Drive, Irvine, CA 92618.

The system 10 can further include an irrigation source (not illustrated) configured to provide irrigation fluid to the catheter 14. The workstation 55 can be configured to control the irrigation source to provide irrigation at the distal end 28 of the catheter 14.

Figure 2A is an illustration of a first example basket assembly 100 having multiple spines 110a-f and electrodes 40 coupled to the spines 110a-f. The spines 110a-f are distributed among a first frame loop 111, a second frame loop 112, and a third frame loop 113. The spines 110a-f are configured to expand away from a longitudinal axis 86 of the end effector to form a basket shape. The basket shape can be approximately spherical, approximately oblate-spheroid, or other suitable shape as understood by a person skilled in the art.

The first frame loop 111 includes a first pair of spines 110a, 110d that are disposed across from each other with respect to the longitudinal axis 86. The second frame loop 112 is distinct from the first frame loop 111 and includes a second pair of spines 110b, 110c that are disposed on a first side of the first frame loop 111. The third frame loop 113 is distinct from the first frame loop 111, and the second frame loop 112 and includes a third pair of spines 110f, 110e that are disposed on a second side of the first frame loop 111. The first frame loop 111, second frame loop 112, and third frame loop 113 converge at a distal end 104 of the basket assembly 100.

The first example basket assembly 100 can be configured to join with the shaft 84 via a retention hub 90 to form a catheter 14 as disclosed in relation to Figure 1 or otherwise joined to the shaft 84 as understood by a person skilled in the pertinent art. The electrodes 40 can be configured for navigation, mapping, and/or ablation as disclosed in relation to Figure 1. Each spine 110a-f can include a support frame, an insulator electrically insulating the support frame from electrodes 40, electrical conductors in electrical communication with the electrodes 40, and other compatible features such as disclosed in relation to Figure 1 and otherwise understood by a person with pertinent skill in the art.

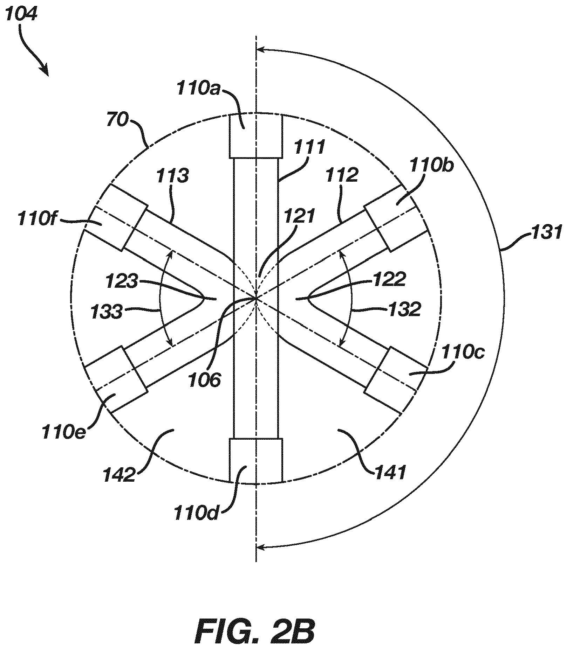

Figure 2B is an illustration of a distal end 104 view of the first example basket assembly 100. The first frame loop 111 includes a distal portion 121 approximate the distal end 104 of the end effector which traverses the longitudinal axis 86. The first frame loop 111 bisects the basket assembly 100 so that the first side (right side in Figure 2B) on which the second frame loop 112 is primarily disposed is opposite the second side (left side in Figure 2B) on which the third frame loop 113 is primarily disposed. Each of the second frame loop 112 and the third frame loop 113 respectively include an angled portion 122, 123 at the distal end 104 of the basket assembly 100 end effector. The angled portion 122, 123 of each of the second and third frame loop can be coupled to the distal portion 121 of the first frame loop 111.

Spines 110a-f are disposed symmetrically about the longitudinal axis 86 such that, collectively, spines of the first pair of spines 110a, 110d of the first frame loop 111, the second pair of spines 110b, 110c of the second frame loop 112, and the third pair of spines 1 10f, 110e of the third frame loop 113 are disposed symmetrically about the longitudinal axis 86.

The first pair of spines 110a, 110d are disposed at an angle 131 of approximately 180° from each other with respect to an imaginary circle 70 about the longitudinal axis 86. The second pair of spines 110b, 110c are disposed at an angle 132 of approximately 60° from each other with respect to the imaginary circle 70. The third pair of spines 110e, 110f are disposed at an angle 133 of approximately 60° from each other with respect to the imaginary circle 70.

Each angled portion 122, 123 of the second frame loop 112 and the third frame loop 113 overlaps the distal portion 121 of the first frame loop 111. As illustrated, the distal portion 121 of the first frame loop 111 is distal of the angled portions 122, 123. Configured as such, the distal portion 121 of the first frame loop 111 may shield the angled portions 122, 123 from contacting tissue, thereby providing an atraumatic distal end 104 to the basket assembly 100. As will be appreciated by a person skilled in the pertinent art, one or both of the angled portions 122, 123 can be positioned distal of the distal portion 121 of the first frame loop 111, and the basket assembly 100 can be otherwise configured to provide an atraumatic distal end 104, for instance, by providing an atraumatic shape at one or both of the angled portions 122, 123, by covering the distal end 104 of the basket assembly 100 with an atraumatic cover or coating, etc.

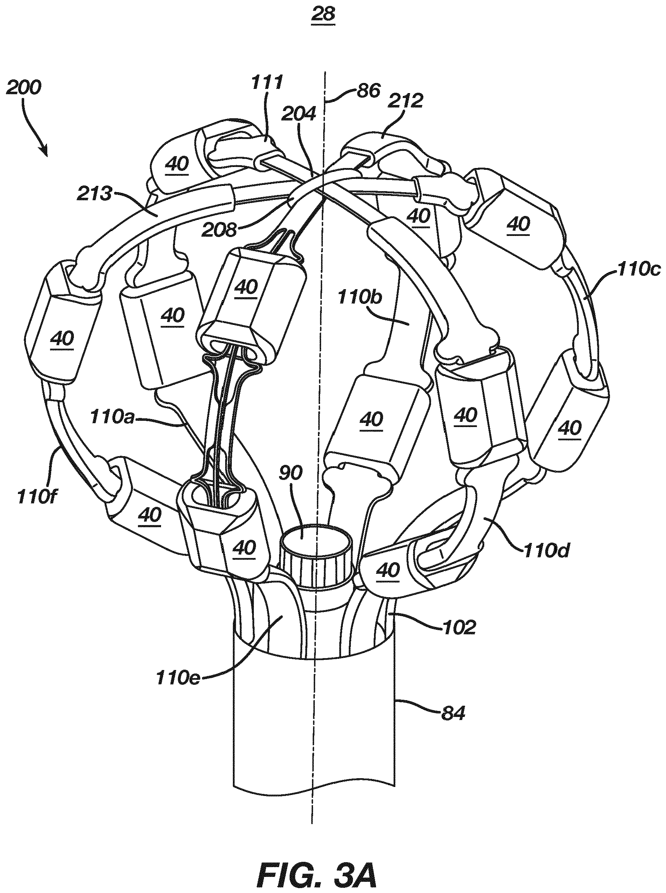

Figure 3A is an illustration of a second example basket assembly 200 having multiple spines 1 10a-f and electrodes 40 coupled to the spines 1 10a-f. The spines 1 10a-f and electrodes 40 are configured similarly to the spines 110a-f and electrodes 40 disclosed in relation to Figure 2A. The electrodes 40 can be configured for navigation, mapping, and/or ablation as disclosed in relation to Figure 1. Each spine 110a-f can include a support frame, an insulator electrically insulating the support frame from electrodes 40, electrical conductors in electrical communication with the electrodes 40, and other compatible features such as disclosed in relation to Figure 1 and otherwise understood by a person with pertinent skill in the art.

The spines 1 10a-f are distributed among a first frame loop 111, a second frame loop 212, and a third frame loop 213. The spines 110a-f are configured to expand away from a longitudinal axis 86 of the end effector to form a basket shape. The basket shape can be approximately spherical, approximately oblate-spheroid, or other suitable shape as understood by a person skilled in the art.

The first frame loop 111 includes a first pair of spines 1 10a, 110d that are disposed across from each other with respect to the longitudinal axis 86. The second frame loop 212 is distinct from the first frame loop 111 and includes a second pair of spines 110b, 110c that are disposed on a first side of the first frame loop 111. The third frame loop 213 is distinct from the first frame loop 111, and the second frame loop 212 and includes a third pair of spines 110f, 110e that are disposed on a second side of the first frame loop 211. The first frame loop 211, second frame loop 212, and third frame loop 113 converge at the distal end 204 of the second example basket assembly 200.

The second example basket assembly 200 can be configured to join with the shaft 84 via a retention hub 90 to form a catheter 14 as disclosed in relation to Figure 1 or otherwise joined to the shaft 84 as understood by a person skilled in the pertinent art.

The second example basket assembly 200 is similar to the first example basket assembly illustrated in Figures 2A and 2B except that the distal end 204 of the second example basket assembly 200 is configured differently than the distal end 104 of the first example basket assembly 100.

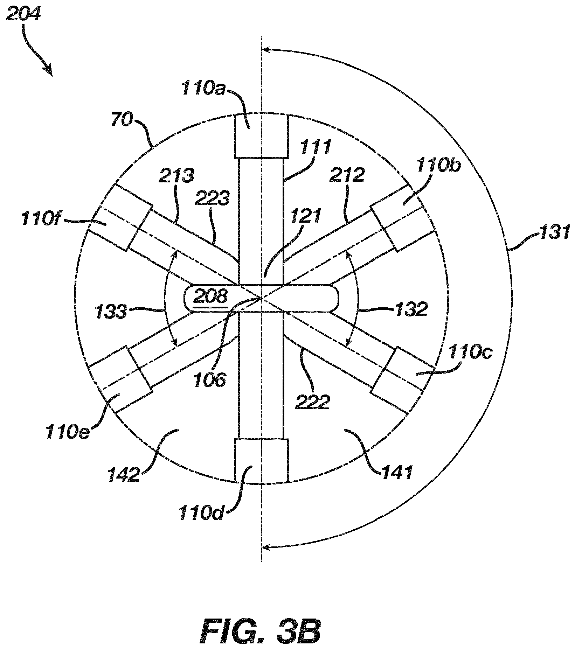

Figure 3B is an illustration of the distal end 204 of the second example basket assembly 200. The first frame loop 111 includes a distal portion 121 approximate the distal end 204 of the end effector which traverses the longitudinal axis 86 similar to the first frame loop 111 of the first example basket assembly 100. The first frame loop 111 bisects the second example basket assembly 200 so that the first side (right side in Figure 3B) on which the second frame loop 212 is primarily disposed is opposite the second side (left side in Figure 3B) on which the third frame loop 213 is primarily disposed. Each of the second frame loop 212 and the third frame loop 213 respectively include an angled portion 222, 223 at the distal end 204 of the basket assembly 200 end effector. Each angled portion 222, 223 abuts the distal portion 121 of the first frame loop rather than overlapping the distal portion 121 of the first frame loop as in the first example basket assembly 100.

The angled portion 222, 223 of each of the second and third frame loop can be coupled to the distal portion 221 of the first frame loop 211. The second example basket assembly 200 includes a retainer 208. Although not illustrated in Figures 2A and 2B, the first example basket assembly 100 can also include the retainer 208. The retainer 208 is illustrated as a band, but can have other alternative configurations as understood by a person skilled in the pertinent art, such as a circular hub. The retainer 208 can be configured to provide an atraumatic distal end 204 of the second example basket assembly 200 (or first example basket assembly 100). The second example basket assembly 200 can be otherwise configured to provide an atraumatic distal end 204, for instance, by providing an atraumatic shape at one or both of the angled portions 222, 223, by covering the distal end 204 of the basket assembly 200 with an atraumatic cover or coating, etc.

Spines 110a-f are disposed symmetrically about the longitudinal axis 86 such that, collectively, spines of the first pair of spines 110a, 110d of the first frame loop 111, the second pair of spines 110b, 110c of the second frame loop 212, and the third pair of spines 1 10f, 110e of the third frame loop 213 are disposed symmetrically about the longitudinal axis 86.

The first pair of spines 110a, 110d are disposed at an angle 131 of approximately 180° from each other with respect to an imaginary circle 70 about the longitudinal axis 86. The second pair of spines 110b, 110c are disposed at an angle 132 of approximately 60° from each other with respect to the imaginary circle 70. The third pair of spines 1 10e, 110f are disposed at an angle 133 of approximately 60° from each other with respect to the imaginary circle 70.

Each angled portion 222, 223 of the second frame loop 212 and the third frame loop 213 abuts the distal portion 121 of the first frame loop 111.

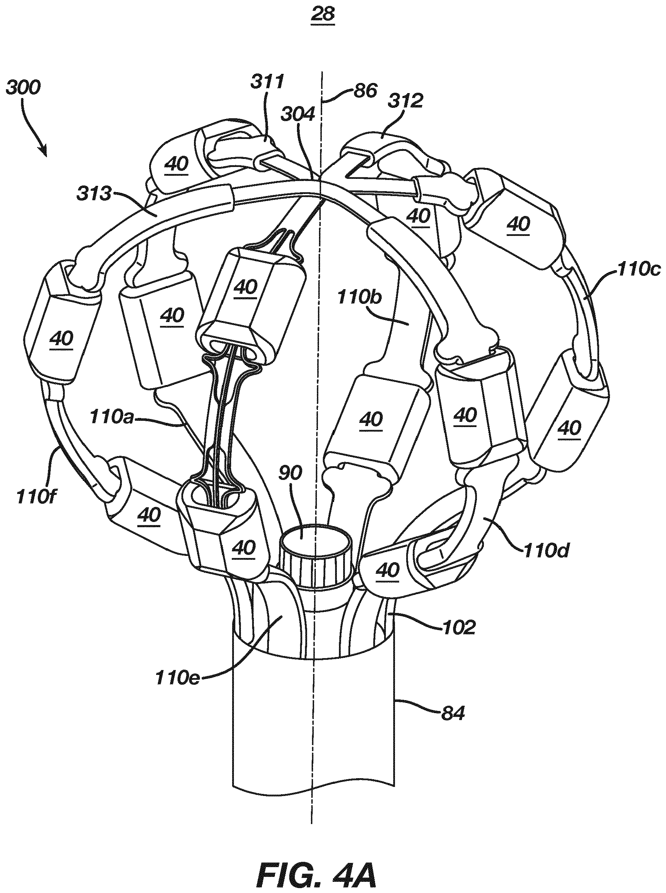

Figure 4A is an illustration of a third example basket assembly 300 having multiple spines 110a-f and electrodes 40 coupled to the spines 1 10a-f. The spines 110a-f and electrodes 40 are configured similarly to the spines 110a-f and electrodes 40 disclosed in relation to Figure 2A. The electrodes 40 can be configured for navigation, mapping, and/or ablation as disclosed in relation to Figure 1. Each spine 110a-f can include a support frame, an insulator electrically insulating the support frame from electrodes 40, electrical conductors in electrical communication with the electrodes 40, and other compatible features such as disclosed in relation to Figure 1 and otherwise understood by a person with pertinent skill in the art.

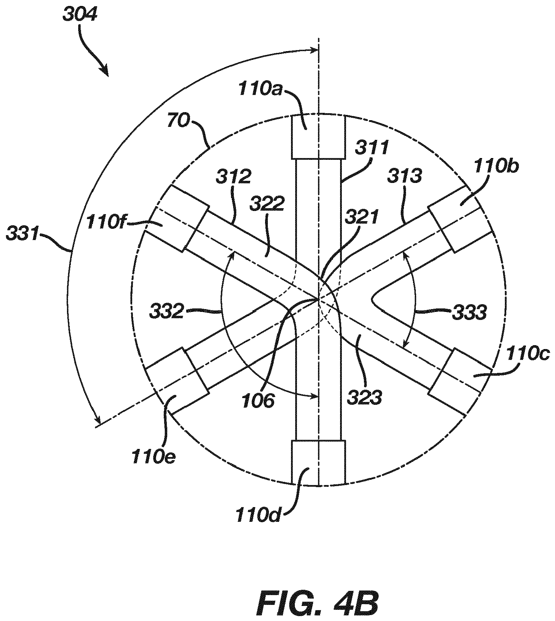

Figure 4B is an illustration of a distal end 304 of the third example basket assembly 300. Referring collectively to Figures 4A and 4B, the spines 110a-f are distributed among a first frame loop 311, a second frame loop 312, and a third frame loop 313. The spines 110a-f are configured to expand away from a longitudinal axis 86 of the end effector to form a basket shape. The basket shape can be approximately spherical, approximately oblate-spheroid, or other suitable shape as understood by a person skilled in the art.

The first frame loop 311 includes a first pair of spines 110a, 110e and a first angled portion 321 approximate the distal end 304 of the end effector. The second frame loop 312 is distinct from the first frame loop 311 and includes a second pair of spines 1 10d, 110f and a second angled portion 322 approximate the distal end 304 of the end effector such that the second angle portion 322 overlaps the first angled portion 321, an inner spine 110e of the first pair of spines 110a, 110e is positioned between spines of the second pair of spines 1 10d, 1 10f, and an inner spine 110f of the second pair of spines 110d, 110f is positioned between spines the first pair of spines 110a, 110e. The third frame loop 313 is distinct from the first frame loop 311 and the second frame loop 312 and includes a third pair of spines 1 10b, 110c and a third angled portion 323 approximate the distal end 304 of the end effector such that spines of the third pair of spines 110b, 110c are each disposed between outer spines 110a, 110d of the first pair of spines and the second pair of spines.

The second example basket assembly 200 can be configured to join with the shaft 84 via a retention hub 90 to form a catheter 14 as disclosed in relation to Figure 1 or otherwise joined to the shaft 84 as understood by a person skilled in the pertinent art.

The first angled portion 321and/or the second angled portion 322 can overlap the third angled portion 323 approximate the distal end 304 of the end effector to provide an atraumatic distal end 304. The angled portions 321, 322, 323 of each of the first, second, and third frame loops 311, 312, 313 can be coupled to each other. Although not illustrated in Figures 4A and 4B, the third example basket assembly 300 can also include a retainer similar to the retainer 208 illustrated in Figures 3A and 3B. The retainer 208 is illustrated as a band, but can have other alternative configurations as understood by a person skilled in the pertinent art, such as a circular hub. The retainer 208 can be configured to provide an atraumatic distal end 304 of the third example basket assembly 300. The third example basket assembly 300 can be otherwise configured to provide an atraumatic distal end 304, for instance, by providing an atraumatic shape at one or more of the angled portions 321, 322, 323, by covering the distal end 304 of the basket assembly 200 with an atraumatic cover or coating, etc.

Spines 110a-f are disposed symmetrically about the longitudinal axis 86 such that, collectively, spines of the first pair of spines 110a, 110e of the first frame loop 311, the second pair of spines 110d, 110f of the second frame loop 312, and the third pair of spines 110b, 110c of the third frame loop 313 are disposed symmetrically about the longitudinal axis 86.

The first pair of spines 110a, 110e are disposed at an angle 331 of approximately 120° from each other with respect to an imaginary circle 70 about the longitudinal axis 86. The second pair of spines 1 10d, 110f are disposed at an angle 332 of approximately 120° from each other with respect to the imaginary circle 70. The third pair of spines 110b, 110c are disposed at an angle 333 of approximately 60° from each other with respect to the imaginary circle 70.

The outer spine 110a of the first pair of spines can be disposed opposite the outer spine 110d of the second pair of spines with respect to the longitudinal axis 86. The inner spines 110e, 110f of the first and second pairs of spines can be positioned opposite a respective spine of the third pair of spines 1 10b, 110c.

Figure 5A is an illustration of a fourth example basket assembly 400 having an inner electrode assembly 420 in a contacting configuration and an outer electrode assembly 420. Electrodes 40b, 40a of the inner electrode assembly 420 and outer electrode assembly 410 are coupled to spines 110a-f. The spines 110a-f and electrodes 40a, 40b are configured similarly to the spines 110a-f and electrodes 40 disclosed in relation to Figure 2A. The electrodes 40a, 40b can be configured for navigation, mapping, and/or ablation as disclosed in relation to the electrodes 40 of Figure 1. Each spine 110a-f can include a support frame, an insulator electrically insulating the support frame from electrodes 40a, 40b, electrical conductors in electrical communication with the electrodes 40a, 40b, and other compatible features such as disclosed in relation to Figure 1 and otherwise understood by a person with pertinent skill in the art.

The outer electrode assembly 410 includes a first plurality of spines 1 10a, 1 10c, 110e and a first plurality of electrodes 40a. The first plurality of electrodes 40a are coupled to each of the first plurality of spines 110a, 110c, 110e. The inner electrode assembly 420 includes a second plurality of spines 110b, 110d, 110f and a second plurality of electrodes 40b. The second plurality of electrodes 40b are coupled to each of the second plurality of spines 11 0b, 110d, 110f.

The first plurality of spines 110a, 110c, 110e are configured to expand from the longitudinal axis 86 to form a basket shape, and the second plurality of spines 110b, 110d, 110f are configured to expand from the longitudinal axis 86 to form a basket shape. The inner and outer electrode assemblies 420, 410 can collectively form a basket shape. The basket shape can be approximately spherical, approximately oblate-spheroid, or other suitable shape as understood by a person skilled in the art.

The inner electrode assembly 420 is configured to move between a non-contacting configuration and a contacting configuration. Figure 5A illustrates the inner electrode assembly 420 in the contacting configuration. In the contacting configuration, the second plurality of electrodes 40b are positioned to contact tissue. The second plurality of spines 110b, 1 10d, 110f are out of alignment with the first plurality of spines 110a, 110c, 110e in the contacting configuration.

Figure 5B is an illustration of a spine 110d of the inner electrode assembly 420 aligned with a spine 110c of the outer electrode assembly 410 when the inner electrode assembly 420 is in the non-contacting configuration. The second plurality of spines 110b, 110d, 110f are in alignment with the first plurality of spines 110a, 11 0c, 1 10e in the non-contacting configuration. In the non-contacting configuration, the second plurality of electrodes 40b are inhibited, by the outer electrode assembly 410, from contacting tissue.

Referring collectively to Figures 5A and 5B, the inner electrode assembly 420 can be configured to move between the non-contacting configuration (Figure 5B) and the contacting configuration (Figure 5A) by virtue of one or both of the inner and outer electrode assemblies 420, 410 rotating about the longitudinal axis 86. For instance, the fourth example basket assembly 400 can be configured in one of three ways to move the inner electrode assembly 420 between the non-contacting configuration and the contacting configuration: (1) the inner electrode assembly 420 can rotate about the longitudinal axis 86 while the outer electrode assembly 410 remains stationary; (2) the outer electrode assembly 410 can rotate about the longitudinal axis 86 while the inner electrode assembly 420 remains stationary; or (3) both the inner electrode assembly 420 and the outer electrode assembly 410 can rotate about the longitudinal axis 86.

The outer electrode assembly 410 can include a first unitary tripodic structure including the first plurality of spines 110a, 11 0c, 110e such that the first plurality of spines has exactly three spines. The inner electrode assembly 420 can include a second unitary tripodic structure including the second plurality of spines 110b, 110d, 110f such that the second plurality of spines has exactly three spines. Each tripodic structure can be formed from a respective planar sheet of material that includes three linear spines converging at a respective central spine intersection 406. Each spine of each tripodic structure can include a respective end disposed at a proximal end of the fourth example basket assembly 400. The central spine intersection 406 of each tripodic structure can be positioned on the longitudinal axis 86 at a distal end of the end effector.

Each electrode 40a, 40b of the first plurality of electrodes 40a and of the second plurality of electrodes 40b can define a lumen through the electrode so that each spine 110a-f extends through the lumen of each of the electrodes. The electrodes 40a, 40b can be shaped similar to as illustrated in Figure 11, variations thereof, and alternatives thereto as understood by a person skilled in the pertinent art. Electrodes of the first plurality of electrodes 40a can be configured to deliver electrical pulses for irreversible electroporation. The pulses can have a peak voltage of at least 900 volts (V). Electrodes of the second plurality of electrodes 40b can be configured to map cardiac electrical signals through tissue.

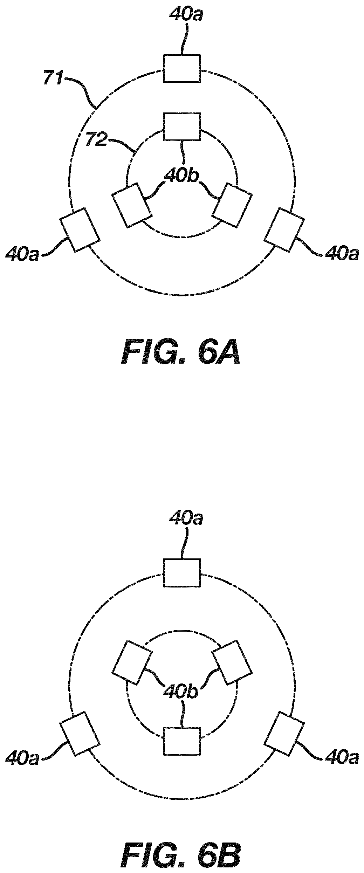

Figure 6A is an illustration of the arrangement of electrodes 40a, 40b of the fourth example basket assembly 400 with the inner electrode assembly 420 in the non-contacting configuration.

Figure 6B is an illustration of the arrangement of electrodes 40a, 40b of the fourth example basket assembly 400 with the inner electrode assembly 420 in the contacting configuration.

In both Figures 6A and 6B, an inner imaginary circle 72 is drawn to show the circular arrangement of the second plurality of electrodes 40b, and an outer imaginary circle 71 is drawn to show the circular arrangement of the first plurality of electrodes 40a.

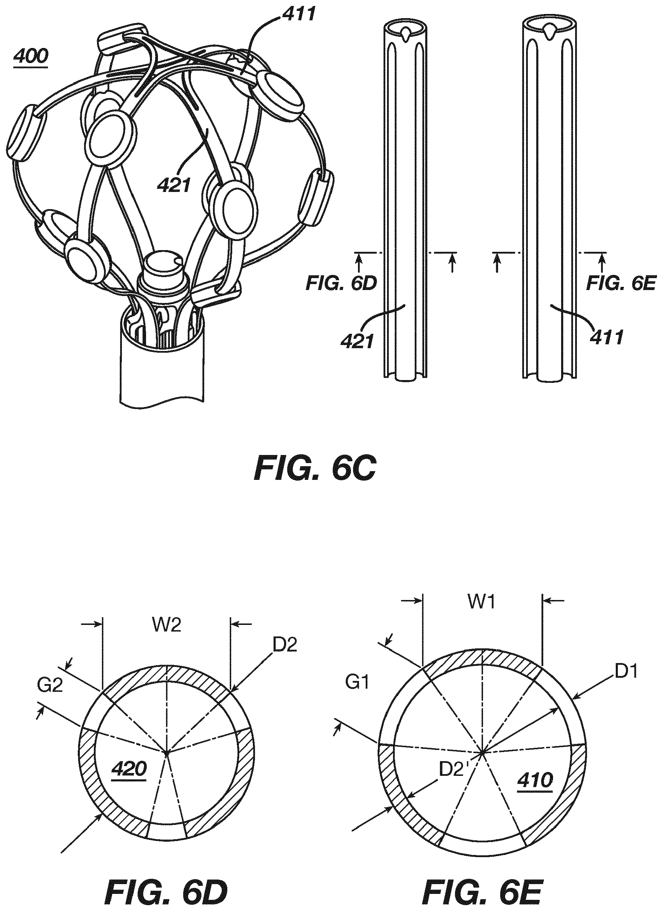

Figure 6C shows an exemplary basket catheter 400 with two formed tubular stocks 421 and 411. When each respective tubular stock 411, 421 is expanded, they become a respective three-spined basket. Electrodes 40a, 40b, conductors, and other features can be affixed to the tube stocks 421, 411 to form the inner electrode assembly 420 and outer electrode assembly 410.

Figure 6D shows the cross-sectional view of the tubular stock 421 of the inner electrode assembly 420 as indicated in Figure 6C. The tubular stock 421 has an outer diameter D2 and is cut to have spines with a width W2 and a gap G2 between spines. The spine width W2 is approximately 72% of the outer diameter D2. The gap G2 is approximately 24% of the diameter D2 or approximately 33% of the width W2 of the spine.

Figure 6E shows the cross-sectional view of the tubular stock 411 of the outer electrode assembly 410 as indicated in Figure 6C. The tubular stock 411 has an outer diameter D1 and is cut to have spines with a width W1 and a gap G1 between spines. The spine width W1 is approximately equal to the spine width W2 of the inner tubular stock 421. The spine width W1 is approximately 57% of the outer diameter D1. The gap G1 is approximately 43% of the outer diameter D1 or approximately 75% of the width W1 of the spine. The outer tubular stock 411 has an inner diameter D2' that is approximately equal to the outer diameter D2 of the inner tube stock 421. When the inner tube stock 421 and outer tube stock 411 are expanded into baskets, the distal tip hole 412 of the outer tube stock 411 can be approximately the same diameter of the outer diameter D2 of the inner tube stock 421.

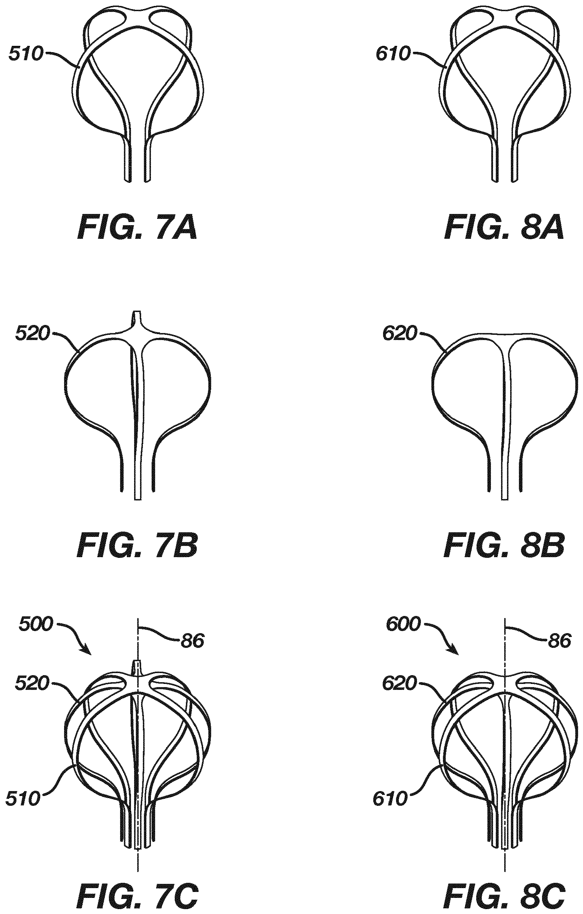

Figure 7A is an illustration of a first unitary structure 510 of a fifth example basket assembly 500. The first unitary structure 510 includes four spines, and can be formed from a planar sheet of material that includes four linear spines converging at a central spine intersection.

Figure 7B is an illustration of a second unitary structure 520 of the fifth example basket assembly 500. The second unitary structure 520 is distinct from the first unitary structure 510 and includes four spines. The second unitary structure 520 can be shaped identically as the first unitary structure 510, but need not be. The second unitary structure 520 can be formed from a planar sheet of material that includes the four spines converging at a central spine intersection.

Figure 7C is an illustration of the structures 510, 520 in Figure 7A and 7B assembled to form the support frame of fifth example basket assembly 500. The fifth example basket assembly 500 can further include electrodes coupled to each spine of the first unitary structure 510 and the second unitary structure 520. The electrodes can be configured similarly to the electrodes 40 disclosed in relation to Figure 1 and in relation to Figure 11. Each electrode can define a lumen through the electrode so that each spine extends through the lumen of each electrode. The electrodes can be configured to deliver electrical pulses for irreversible electroporation, the pulses including a peak voltage of at least 900 volts (V).

The spines of the first unitary structure 510 and the second unitary structure 520 can be configured to expand from a longitudinal axis of the end effector to collectively form a basket shape. The basket shape can be approximately spherical or approximately oblate-spheroid. The spines of the first unitary structure 510 and the second unitary structure 520 can be positioned symmetrically about the longitudinal axis 86.

Each spine of each unitary structure 510, 520 can include a respective connected end disposed at a proximal end of the fifth example basket assembly 500. The central spine intersection of each unitary structure 510, 520 can be positioned on the longitudinal axis 86 at a distal end of the basket assembly 500.

Figure 8A is an illustration of a first unitary structure 610 of a sixth example basket assembly 600. The first unitary structure 610 includes four spines, and can be formed from a planar sheet of material that includes four linear spines converging at a central spine intersection.

Figure 8B is an illustration of a second unitary structure 620 of the sixth example basket assembly 600. The second unitary structure 620 is distinct from the first unitary structure 610 and includes three spines. The second unitary structure 620 can be formed from a planar sheet of material that includes the three spines converging at a central spine intersection.

Figure 8C is an illustration of the structures 610, 620 in Figure 8A and 8B assembled to form the support frame of sixth example basket assembly 600. The sixth example basket assembly 600 can further include electrodes coupled to each spine of the first unitary structure 610 and the second unitary structure 620. The electrodes can be configured similarly to the electrodes 40 disclosed in relation to Figure 1 and in relation to Figure 11. Each electrode can define a lumen through the electrode so that each spine extends through the lumen of each electrode. The electrodes can be configured to deliver electrical pulses for irreversible electroporation, the pulses including a peak voltage of at least 900 volts (V).

The spines of the first unitary structure 610 and the second unitary structure 620 can be configured to expand from a longitudinal axis of the end effector to collectively form a basket shape. The basket shape can be approximately spherical or approximately oblate-spheroid. The spines of the first unitary structure 610 and the second unitary structure 620 can be positioned symmetrically about the longitudinal axis 86.

Each spine of each unitary structure 610, 620 can include a respective connected end disposed at a proximal end of the sixth example basket assembly 600. The central spine intersection of each unitary structure 610, 620 can be positioned on the longitudinal axis 86 at a distal end of the basket assembly 600.

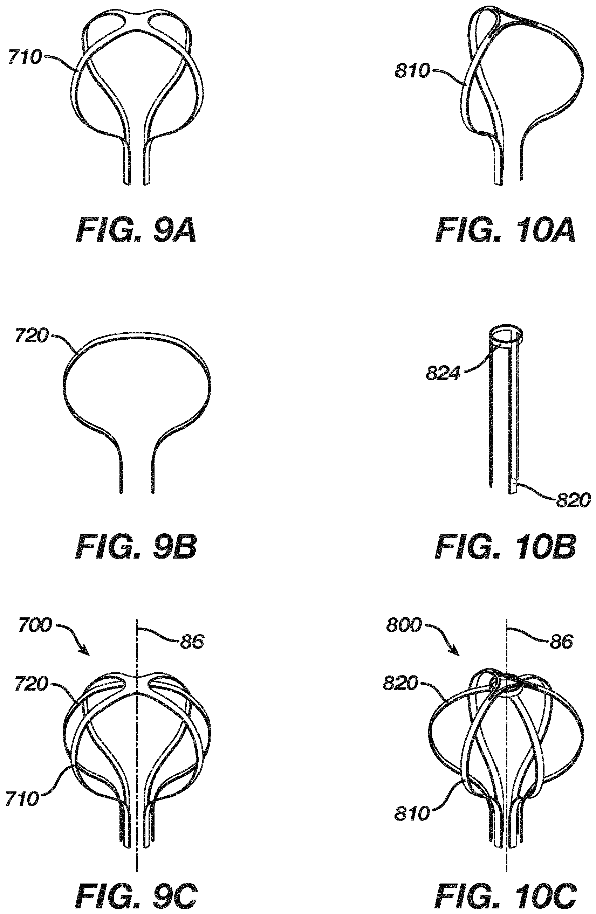

Figure 9A is an illustration of a first unitary structure 710 of a seventh example basket assembly 700. The first unitary structure 710 includes four spines, and can be formed from a planar sheet of material that includes four linear spines converging at a central spine intersection.

Figure 9B is an illustration of a second unitary structure 720 of the seventh example basket assembly 700. The second unitary structure 720 is distinct from the first unitary structure 710 and includes two spines. The second unitary structure 720 can be shaped from a single elongated spine shaped to form a loop. The second unitary structure 720 can be formed from a planar sheet of material. The central point of the loop is considered a central spine intersection.

Figure 9C is an illustration of the structures 710, 720 in Figure 9A and 9B assembled to form the support frame of seventh example basket assembly 700. The second unitary structure 720 can include a loop extending across the central spine intersection of the first unitary structure 710 such that two spines of the first unitary structure 710 are disposed on a first side of the loop 720 and two spines of the first unitary structure 710 are disposed on a second side of the loop 720.

The seventh example basket assembly 700 can further include electrodes coupled to each spine of the first unitary structure 710 and the second unitary structure 720. The electrodes can be configured similarly to the electrodes 40 disclosed in relation to Figure 1 and in relation to Figure 11. Each electrode can define a lumen through the electrode so that each spine extends through the lumen of each electrode. The electrodes can be configured to deliver electrical pulses for irreversible electroporation, the pulses including a peak voltage of at least 900 volts (V).

The spines of the first unitary structure 710 and the second unitary structure 720 can be configured to expand from a longitudinal axis of the end effector to collectively form a basket shape. The basket shape can be approximately spherical or approximately oblate-spheroid. The spines of the first unitary structure 710 and the second unitary structure 720 can be positioned symmetrically about the longitudinal axis 86.

Each spine of each unitary structure 710, 720 can include a respective connected end disposed at a proximal end of the seventh example basket assembly 700. The central spine intersection of each unitary structure 710, 720 can be positioned on the longitudinal axis 86 at a distal end of the basket assembly 700.

Figure 10A is an illustration of a first unitary structure 810 of an eighth example basket assembly 800. The first unitary structure 810 includes three spines, and can be formed from a planar sheet of material that includes three linear spines converging at a central spine intersection.

Figure 10B is an illustration of a second unitary structure 820 of the eighth example basket assembly 800. The second unitary structure 820 is distinct from the first unitary structure 810 and includes three spines. The second unitary structure 820 can be formed from a tube of material that includes the at least two spines joined at one end by a ring.

Figure 10C is an illustration of the structures 810, 820 in Figure 10A and 10B assembled to form the support frame of eighth example basket assembly 800.

The eighth example basket assembly 800 can further include electrodes coupled to each spine of the first unitary structure 810 and the second unitary structure 820. The electrodes can be configured similarly to the electrodes 40 disclosed in relation to Figure 1 and in relation to Figure 11. Each electrode can define a lumen through the electrode so that each spine extends through the lumen of each electrode. The electrodes can be configured to deliver electrical pulses for irreversible electroporation, the pulses including a peak voltage of at least 900 volts (V).

The spines of the first unitary structure 810 and the second unitary structure 820 can be configured to expand from a longitudinal axis of the end effector to collectively form a basket shape. The basket shape can be approximately spherical or approximately oblate-spheroid. The spines of the first unitary structure 810 and the second unitary structure 820 can be positioned symmetrically about the longitudinal axis 86.

Each spine of each unitary structure 810, 820 can include a respective connected end disposed at a proximal end of the eighth example basket assembly 800. The central spine intersection of each unitary structure 810, 820 can be positioned on the longitudinal axis 86 at a distal end of the basket assembly 800.

Figure 11 is an illustration of an electrode 40. The electrode defines a lumen 48 through the electrode 40 so that a spine can extends through the lumen 48. The electrode has an outer surface 44 that is exposed to ambient environment and an inner surface 46 within the lumen 48. The electrode 40 can include electrically conductive material (e.g., gold, platinum and palladium (and their respective alloys)). The electrode 40 can have a variety of cross-sectional shapes, curvatures, lengths, lumen number and lumen shapes. The electrode 40 illustrated in Figure 11 and elsewhere herein are offered to illustrate various configurations of electrodes 40 that can be used with the catheter 14 but should not be construed as limiting. One skilled in the art will appreciate that various other configurations of electrodes 40 can be used with the disclosed technology without departing from the scope of this disclosure. The electrodes 40 includes a wire relief 42 forming a recess or depression in the electrode 40 adjacent the lumen 48 for one or more wires to pass through the lumen 48 along with a respective spine 110. Relief 42 can be sized to provide room for a wire of electrode 40 to pass through electrode 40 such that electrode 40 can be in electrical communication with the control console PIU 30 (Figure 1).

Figure 12A is an illustration of a frame loop with an approximately circular cross-section which illustrates a profile of a basket assembly having a basket shape that is approximately spherical. Any of the example basket assemblies 100, 200, 300, 400, 500, 600, 700, 800 can be shaped to have a basket shape that is approximately spherical.

Figure 12B is an illustration of a frame loop with an oblate cross-section which illustrates a profile of a basket assembly having a basket shape that is approximately oblate-spheroid. Any of the example basket assemblies 100, 200, 300, 400, 500, 600, 700, 800 can be shaped to have a basket shape that is approximately oblate-spheroid.

Having shown and described exemplary embodiments of the subject matter contained herein, further adaptations of the methods and systems described herein may be accomplished by appropriate modifications without departing from the scope of the claims. In addition, where methods and steps described above indicate certain events occurring in certain order, it is intended that certain steps do not have to be performed in the order described, but in any order, as long as the steps allow the embodiments to function for their intended purposes. Therefore, to the extent there are variations of the invention, which are within the spirit of the disclosure or equivalent to the inventions found in the claims, it is the intent that this patent will cover those variations as well. Some such modifications should be apparent to those skilled in the art. For instance, the examples, embodiments, geometries, materials, dimensions, ratios, steps, and the like discussed above are illustrative. Accordingly, the claims should not be limited to the specific details of structure and operation set forth in the written description and drawings.

The following clauses list non-limiting embodiments of the disclosure:
Clause 1. An end effector of a catheter, the end effector comprising: a plurality of spines configured to expand away from a longitudinal axis of the end effector to form a basket shape; a first frame loop comprising a first pair of spines of the plurality of spines such that spines of the first pair of spines are disposed across from each other with respect to the longitudinal axis; a second frame loop , distinct from the first frame loop, comprising a second pair of spines of the plurality of spines such that spines of the second pair of spines are disposed on a first side of the first frame loop; a third frame loop, distinct from the first frame loop and the second frame loop, comprising a third pair of spines of the plurality of spines such that spines of the third pair of spines are disposed on a second side of the first frame loop, the second side being opposite the first side; and one or more electrodes coupled to the plurality of spines.
Clause 2. The end effector of clause 1, the first frame loop comprising a distal portion approximate a distal end of the end effector and traversing the longitudinal axis.
Clause 3. The end effector of clause 1 or 2, each of the second frame loop and the third frame loop comprising an angled portion approximate a distal end of the end effector, the angled portion of each of the second and third frame loop being coupled to the first frame loop.
Clause 4. The end effector of any one of clauses 1-3, spines of the plurality of spines being disposed symmetrically about the longitudinal axis.
Clause 5. The end effector of clause 1, spines of the first pair of spines, the second pair of spines, and the third pair of spines being disposed symmetrically about the longitudinal axis.
Clause 6. The end effector of any one of clauses 1-5, the first pair of spines being disposed approximately 180° from each other with respect to an imaginary circle about the longitudinal axis, the second pair of spines being disposed approximately 60° from each other with respect to the imaginary circle, and the third pair of spines being disposed approximately 60° from each other with respect to the imaginary circle.
Clause 7. The end effector of any one of clauses 1-6, the first frame loop comprising a distal portion at a distal end of the end effector and traversing the longitudinal axis, and each of the second frame loop and the third frame loop comprising an angled portion approximate a distal end of the end effector such that each angled portion overlaps the distal portion of the first frame loop.
Clause 8. The end effector of any one of clauses 1-6, the first frame loop comprising a distal portion at a distal end of the end effector and traversing the longitudinal axis, and each of the second frame loop and the third frame loop comprising an angled portion approximate a distal end of the end effector such that each angled portion abuts the distal portion of the first frame loop.
Clause 9. The end effector of clause 8, the second frame loop being disposed entirely on the first side of the first frame loop, and the third frame loop being disposed entirely on the second side of the first frame loop.
Clause 10. The end effector of any one of clauses 1-9, further comprising: a retainer coupling the first frame loop, the second frame loop, and the third frame loop approximate a distal end of the end effector.
Clause 11. The end effector of any one of clauses 1-10, each electrode of the one or more electrodes defining a lumen through the electrode so that each spine of the plurality spines extends through the lumen of each of the one or more electrodes.
Clause 12. The end effector of any one of clauses 1-11, wherein the basket shape is approximately spherical.
Clause 13. The end effector of any one of clauses 1-11, wherein the basket shape is approximately oblate-spheroid.
Clause 14. The end effector of any one of clauses 1-13, wherein the one or more electrodes are configured to deliver electrical pulses for irreversible electroporation, the pulses including a peak voltage of at least 900 volts (V).
Clause 15. An end effector of a catheter, the end effector comprising: a plurality of spines configured to expand from a longitudinal axis of the end effector to form a basket shape; a first frame loop comprising a first pair of spines of the plurality of spines and a first angled portion approximate a distal end of the end effector; a second frame loop, distinct from the first frame loop, comprising a second pair of spines of the plurality of spines and a second angled portion approximate the distal end of the end effector such that the second angle portion overlaps the first angled portion, an inner spine of the first pair of spines is positioned between spines of the second pair of spines, and an inner spine of the second pair of spines is positioned between spines the first pair of spines; a third frame loop, distinct from the first frame loop and the second frame loop, comprising a third pair of spines of the plurality of spines and a third angled portion approximate the distal end of the end effector such that spines of the third pair of spines are each disposed between outer spines of the first pair of spines and the second pair of spines; and one or more electrodes coupled to the plurality of spines.
Clause 16. The end effector of clause 15, spines of the plurality of spines being disposed symmetrically about the longitudinal axis.
Clause 17. The end effector of clause 15 or 16, spines of the first pair of spines, the second pair of spines, and the third pair of spines being disposed symmetrically about the longitudinal axis.
Clause 18. The end effector of any one of clauses 15-17, the first pair of spines being disposed approximately 120° from each other with respect to an imaginary circle about the longitudinal axis, the second pair of spines being disposed approximately 120° from each other with respect to the imaginary circle, and the third pair of spines being disposed approximately 60° from each other with respect to the imaginary circle.
Clause 19. The end effector of any one of clauses 15-18, the outer spine of the first pair of spines being disposed opposite the outer spine of the second pair of spines with respect to the longitudinal axis.
Clause 20. The end effector of any one of clauses 15-19, wherein the first angled portion and/or the second angled portion overlaps the third angled portion approximate the distal end of the end effector.
Clause 21. The end effector of any one of clauses 15-20, each electrode of the one or more electrodes defining a lumen through the electrode so that each spine of the plurality spines extends through the lumen of each of the one or more electrodes.
Clause 22. The end effector of any one of clauses 15-21, wherein the basket shape is approximately spherical.
Clause 23. The end effector of any one of clauses 15-21, wherein the basket shape is approximately oblate-spheroid.
Clause 24. The end effector of any one of clauses 15-23, wherein the one or more electrodes are configured to deliver electrical pulses for irreversible electroporation, the pulses including a peak voltage of at least 900 volts (V).
Clause 25. An end effector of a catheter, the end effector comprising: an outer electrode assembly comprising a first plurality of spines and a first plurality of electrodes, the first plurality of spines being configured to expand from a longitudinal axis of the end effector to form a basket shape, and the first plurality of electrodes being coupled to each of the first plurality of spines; and an inner electrode assembly comprising a second plurality of electrodes, the inner electrode assembly being configured to move between a non-contacting configuration and a contacting configuration such that in the non-contacting configuration, the second plurality of electrodes are inhibited, by the outer electrode assembly, from contacting tissue, and such that in the contacting configuration, the second plurality of electrodes are positioned to contact tissue.
Clause 26. The end effector of clause 25, the outer electrode assembly comprising a first unitary tripodic structure such that the first plurality of spines consists of three spines.
Clause 27. The end effector of clause 26, the inner electrode assembly comprising a second unitary tripodic structure such that the second plurality of spines consists of three spines and the second unitary tripodic structure is rotatable to be in alignment with the first unitary tripodic structure in the non-contacting configuration and to be out of alignment with first unitary tripodic structure in the contacting configuration.
Clause 28. The end effector of clause 27, each tripodic structure formed from a respective planar sheet of material that includes three linear spines converging at a respective central spine intersection, each spine of each tripodic structure including a respective end disposed at a proximal end of the end effector, and the central spine intersection of each tripodic structure being positioned on the longitudinal axis at a distal end of the end effector.
Clause 29. The end effector of any one of clauses 25-28, the inner electrode assembly comprising a second plurality of spines, the second plurality of electrodes being coupled to the second plurality of spines, the second plurality of spines being in alignment with the first plurality of spines in the non-contacting configuration, and the second plurality of spines being out of alignment with the first plurality of spines in the contacting configuration.
Clause 30. The end effector of any one of clauses 25-29, each electrode of the first plurality of electrodes and of the second plurality of electrodes defining a lumen through the electrode so that each spine of the plurality spines extends through the lumen of each of the one or more electrodes.
Clause 31. The end effector of any one of clauses 25-30, wherein the basket shape is approximately spherical.
Clause 32. The end effector of any one of clauses 25-30, wherein the basket shape is approximately oblate-spheroid.
Clause 33. The end effector of any one of clauses 25-32, wherein electrodes of the first plurality of electrodes are configured to deliver electrical pulses for irreversible electroporation, the pulses including a peak voltage of at least 900 volts (V).
Clause 34. The end effector of any one of clauses 25-33, wherein electrodes of the second plurality of electrodes are configured to map cardiac electrical signals through tissue.
Clause 35. An end effector of a catheter, the end effector comprising: an expandable basket assembly comprising: a first unitary structure comprising four spines and being formed from a planar sheet of material that includes four linear spines converging at a central spine intersection, a second unitary structure, distinct from the first unitary structure, comprising at least two spines, and a plurality of electrodes coupled to each spine of the first unitary structure and the second unitary structure, the spines of the first unitary structure and the second unitary structure being configured to expand from a longitudinal axis of the end effector to collectively form a basket shape.
Clause 36. The end effector of clause 35, the secondary unitary structure being formed from a planar sheet of material that includes the at least two spines converging at a central spine intersection, each spine of each unitary structure including a respective connected end disposed at a proximal end of the end effector, and the central spine intersection of each unitary structure being positioned on the longitudinal axis at a distal end of the end effector.
Clause 37. The end effector of clause 35, the second unitary structure being formed from a tube of material that includes the at least two spines joined at one end by a ring.
Clause 38. The end effector of any one of clauses 35-36, the at least two spines consisting of four spines.
Clause 39. The end effector of any one of clauses 35-36, the at least two spines consisting of three spines.
Clause 40. The end effector of any one of clauses 35-36, the at least two spines consisting of two spines.
Clause 41. The end effector of clause 40, the second unitary structure comprising a loop extending across the central spine intersection of the first unitary structure such that two spines of the first unitary structure are disposed on a first side of the loop and two spines of the first unitary structure are disposed on a second side of the loop.
Clause 42. The end effector of any one of clauses 35-41 the spines of the first unitary structure and the second unitary structure being positioned symmetrically about the longitudinal axis.
Clause 43. The end effector of any one of clauses 35-42, each electrode of the plurality of electrodes defining a lumen through the electrode so that each spine of the plurality spines extends through the lumen of each of the one or more electrodes.
Clause 44. The end effector of any one of clauses 35-43, wherein the basket shape is approximately spherical.
Clause 45. The end effector of any one of clauses 35-43, wherein the basket shape is approximately oblate-spheroid.
Clause 46. The end effector of any one of clauses 35-45, wherein the one or more electrodes are configured to deliver electrical pulses for irreversible electroporation, the pulses including a peak voltage of at least 900 volts (V).

Aspects of the invention:
1. An end effector of a catheter, the end effector comprising:
   a plurality of spines configured to expand from a longitudinal axis of the end effector to form a basket shape;
   a first frame loop comprising a first pair of spines of the plurality of spines and a first angled portion approximate a distal end of the end effector;
   a second frame loop, distinct from the first frame loop, comprising a second pair of spines of the plurality of spines and a second angled portion approximate the distal end of the end effector such that the second angle portion overlaps the first angled portion, an inner spine of the first pair of spines is positioned between spines of the second pair of spines, and an inner spine of the second pair of spines is positioned between spines the first pair of spines;
   a third frame loop, distinct from the first frame loop and the second frame loop, comprising a third pair of spines of the plurality of spines and a third angled portion approximate the distal end of the end effector such that spines of the third pair of spines are each disposed between outer spines of the first pair of spines and the second pair of spines; and
   one or more electrodes coupled to the plurality of spines.
2. The end effector of aspect 1, spines of the first pair of spines, the second pair of spines, and the third pair of spines being disposed symmetrically about the longitudinal axis.
3. The end effector of aspect 1 or aspect 2,
   the first pair of spines being disposed approximately 120° from each other with respect to an imaginary circle about the longitudinal axis,
   the second pair of spines being disposed approximately 120° from each other with respect to the imaginary circle, and
   the third pair of spines being disposed approximately 60° from each other with respect to the imaginary circle.
4. The end effector of any of aspects 1 to 3, the outer spine of the first pair of spines being disposed opposite the outer spine of the second pair of spines with respect to the longitudinal axis.
5. The end effector of any of aspects 1 to 4, wherein the first angled portion and/or the second angled portion overlaps the third angled portion approximate the distal end of the end effector.
6. The end effector of any of aspects 1 to 5, each electrode of the one or more electrodes defining a lumen through the electrode so that each spine of the plurality spines extends through the lumen of each of the one or more electrodes.
7. The end effector of any of aspects 1 to 6, wherein the basket shape is approximately spherical.
8. The end effector of any of aspects 1 to 7, wherein the one or more electrodes are configured to deliver electrical pulses for irreversible electroporation, the electrical pulses including a peak voltage of at least 900 volts (V).

## Claims

1. An end effector of a catheter, the end effector comprising:
a plurality of spines configured to expand away from a longitudinal axis of the end effector to form a basket shape;
a first frame loop comprising a first pair of spines of the plurality of spines such that spines of the first pair of spines are disposed across from each other with respect to the longitudinal axis;
a second frame loop , distinct from the first frame loop, comprising a second pair of spines of the plurality of spines such that spines of the second pair of spines are disposed on a first side of the first frame loop;
a third frame loop, distinct from the first frame loop and the second frame loop, comprising a third pair of spines of the plurality of spines such that spines of the third pair of spines are disposed on a second side of the first frame loop, the second side being opposite the first side; and
one or more electrodes coupled to the plurality of spines.

2. The end effector of claim 1, the first frame loop comprising a distal portion approximate a distal end of the end effector and traversing the longitudinal axis.

3. The end effector of claim 1 or claim 2, each of the second frame loop and the third frame loop comprising an angled portion approximate a distal end of the end effector, the angled portion of each of the second and third frame loop being coupled to the first frame loop.

4. The end effector of claim 1, spines of the first pair of spines, the second pair of spines, and the third pair of spines being disposed symmetrically about the longitudinal axis.

5. The end effector of any preceding claim,
the first pair of spines being disposed approximately 180° from each other with respect to an imaginary circle about the longitudinal axis,
the second pair of spines being disposed approximately 60° from each other with respect to the imaginary circle, and
the third pair of spines being disposed approximately 60° from each other with respect to the imaginary circle.

6. The end effector of any preceding claim,
the first frame loop comprising a distal portion at a distal end of the end effector and traversing the longitudinal axis, and
each of the second frame loop and the third frame loop comprising an angled portion approximate a distal end of the end effector such that each angled portion overlaps the distal portion of the first frame loop.

7. The end effector of any of claims 1 to 6,
the first frame loop comprising a distal portion at a distal end of the end effector and traversing the longitudinal axis, and
each of the second frame loop and the third frame loop comprising an angled portion approximate a distal end of the end effector such that each angled portion abuts the distal portion of the first frame loop.

8. The end effector of claim 7, the second frame loop being disposed entirely on the first side of the first frame loop, and the third frame loop being disposed entirely on the second side of the first frame loop.

9. The end effector of any preceding claim, further comprising:
a retainer coupling the first frame loop, the second frame loop, and the third frame loop approximate a distal end of the end effector.

10. The end effector of any preceding claim, each electrode of the one or more electrodes defining a lumen through the electrode so that each spine of the plurality spines extends through the lumen of each of the one or more electrodes.

11. The end effector of any preceding claim, wherein the basket shape is approximately spherical.

12. The end effector of any preceding claim, wherein the one or more electrodes are configured to deliver electrical pulses for irreversible electroporation, the electrical pulses including a peak voltage of at least 900 volts (V).
